# EUROPEAN PATENT SPECIFICATION

(11) **EP 2 492 211 B1**
(45) Date of publication and mention of the grant of the patent: **31.07.2013**
(21) Application number: 11155835.9
(22) Date of filing: 24.02.2011
(51) Int. Cl.: B65D 81/05

(54) **Deformable protective support**
Verformbarer Schutzträger
Support protecteur déformable

(43) Date of publication of application: 29.08.2012
(73) Proprietor: Advance kites S.r.l., 25057 Sale Marasino (BS) (IT)
(72) Inventor: Mazzucchelli, Alessandro, 25057 Sale Marasino (Brescia) (IT)
(74) Representative: Lunati, Valerio

(56) References cited:
- GB-A- 2 089 325
- GB-A- 2 433 736
- US-A- 3 968 620
- US-A- 4 640 080
- US-A- 5 246 114

## Description

The present invention relates to a deformable protective support or wrapper of the type pointed out in the preamble of the first claim. A similar support is disclosed in patent document US-A-4640080.

In particular, the invention concerns a protective support or wrapper adaptable to the outer profile of at least part of an object so as to surround it and protect it against impacts, shocks and/or external agents such as sudden changes of temperature and moisture, for example.

It is known that the protective supports presently used can be substantially divided into two classes, the stiff ones and the deformable ones.

The stiff supports vary based on the use they are conceived for, and therefore they differ from each other as to the materials used and the shape, which elements change depending on the object to be protected. These supports are greatly constrained by the object they have to protect and therefore they are hardly adaptable to objects of different conformation.

The second supports, i.e. the deformable ones, do not have a stiff structure thus being easily adaptable to objects having shapes and sizes different from each other.

These deformable supports are usually made up of an outer wrapper, generally of polymeric material, adapted to enable the product to be protected against external agents, and of an inner structure adapted to absorb stresses resulting from an impact.

Therefore, the most important part appears to be the inner structure that, depending on the expected impacts, is usually made of an elastomeric material or solid foams. In detail, these solid foams can be of the open-cell type, if a high deformability is required, or of the closed-cell type if, on the contrary, more capability of resisting the impacts is necessary.

The above mentioned known art has some important drawbacks.

A first problem consists in that these solutions never constitute the optimal choice but merely represent a compromise between the requirement of adapting themselves to the outer profile of the object to be covered and that of bearing high stresses.

Another problem is represented by the not very high degree of deformability of the presently made supports, so that they have a poor adaptability to the profile of the object to be protected. In particular, while use of inner structures made of open-cell foams offers an improved adaptability of the support as compared with those made of other materials, however it does not ensure an appropriate deformation and therefore a sufficient adaptability of the support to the object's profile.

In detail, this imperfect and imprecise coupling between support and object may give rise, in case of an impact, to the creation of too strong stresses in the contact region between support and object that can lead to breaking of the object.

In conclusion, these protective supports are almost useless for storage of brittle products such as bottles.

Under this situation, the technical task underlying the present invention is to conceive a deformable protective support capable of substantially obviating the mentioned drawbacks.

Within the scope of this technical task it is an important aim of the invention to create a protective support characterised both by high deformability and great capability of absorbing impacts.

Another important aim of the invention is to obtain a support of a particularly simple use and reduced cost.

The technical task mentioned and the aims specified are achieved by a deformable protective support as claimed in the appended Claim 1.

Preferred embodiments are highlighted in the sub-claims.

The features and advantages of the invention are hereinafter clarified by the detailed description of a preferred embodiment of the invention, with reference to the accompanying drawings, in which:
**Fig. 1** is a view of the deformable protective support according to the invention;
**Fig. 2a** shows a section of the support in a first configuration; and
**Fig. 2b** shows a second position of the deformable protective support in a second configuration.

With reference to the drawings, the deformable protective support or wrapper according to the invention is generally identified by the reference numeral **1.**

This deformable support is suitable to internally house at least part of an object **10** and protect it both from impacts and from possible external agents, such as moisture and sudden temperature changes that can damage the object itself. This object 10 can therefore consist both of an element of small sizes, such as a bottle or a camera, and of elements of big sizes, a screen for example.

The deformable protective support 1 comprises an outer wrapper **2** defining a housing volume **2a** adapted to hold at least part of the object 10. Fitted in the housing volume 2a is at least one inner wrapper **3** adapted to be disposed in contact with at least the aforesaid part of object 10 and confining at least one operating chamber **3a** internal to the housing volume 2a. Provided in the operating chamber 3a is a plurality of distinct particles **4.**

In detail, the outer wrapper 2 is adapted to define a hermetic housing volume 2a when the support 1 tightly surrounds the object 10, as hereinafter described. It is therefore made of a material impervious to passage of air (airtight material) and preferably of a polymeric and flexible material.

For instance, the outer wrapper 2 is made of Neoprene®, polyvinylchloride or still other materials.

The outer wrapper 2 has a profile adapted to define a housing volume 2a capable of holding the whole portion of the object 10 to be protected. Preferably, object 10 is a brittle object, such as an electronic device, a glass element or others, but said object 10 may even be a portion or a human body or others.

In order to enable introduction of the object into such a cavity, one or more orifices **2b** are provided as well as closing means **2c** adapted to obtain a hermetically sealed closure of said orifices 2b, i.e. a closure adapted to prevent air passage therethrough.

In detail, if the object 10 is only partly housed in the deformable support 1, the outer wrapper 2 abuts against the object 10 at least at one of the orifices 2b and therefore the closing means 2c comprises elastic strips, Velcro or other similar means adapted to shut and fasten the orifices 2b to the object 10 so as to obtain said hermetic closure.

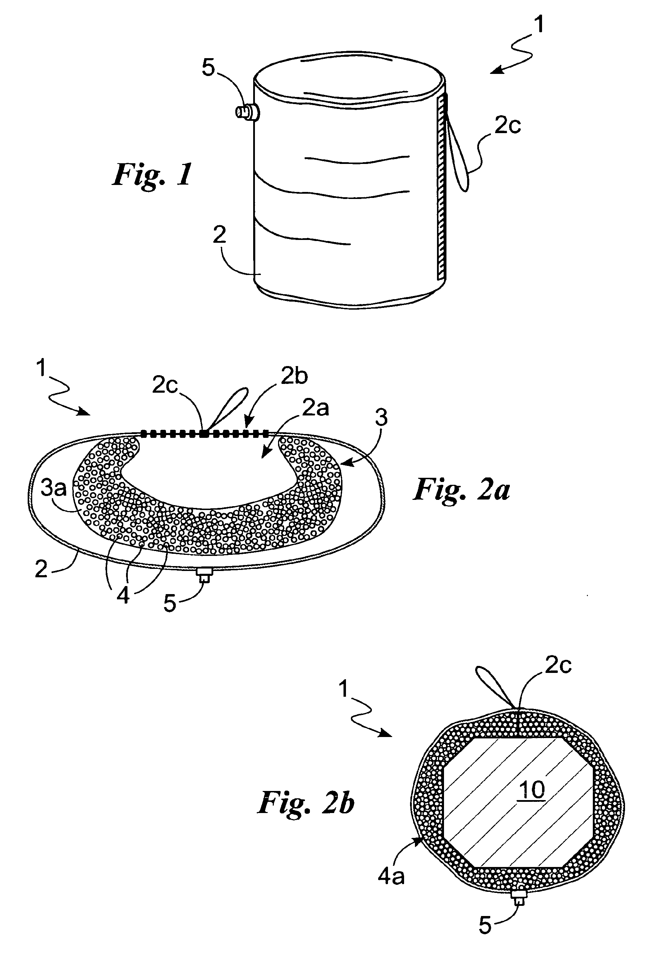

Alternatively, if the object 10 is fully stored in the housing volume 2a, the closing means 2c is adapted to sealingly close the orifice 2b and therefore comprises an impermeable zip (Fig. 1), Velcro or other similar element adapted to perform the aforesaid function.

Inside the housing volume 2a, the deformable support 1 has the inner wrapper adapted to perform the aforesaid function.

Inside the housing volume 2a, the deformable support 1 has the inner wrapper 3 which is possibly partly secured to the outer wrapper 2 by means of glues, seams, Velcro or other similar solutions.

The inner wrapper 3 delimits one or more operating chambers 3a. A single operating chamber 3a can be provided which is disposed between the two mutually secured wrappers 2 and 3.

Alternatively, the single operating chamber 3a is preferably obtained by the inner wrapper 3 alone. In particular, in the example shown in Figs. 2a and 2b, the inner wrapper 3 consists of a single membrane closed upon itself to define an operating chamber. Closure is obtained through seams, glues or others for example, in such a manner as to prevent the distinct particles 4 housed in the inner wrapper 3, from escaping from the operating chamber 3a.

The inner wrapper 3 is adapted to bring the operating chamber 3a into connection for gas passage with the housing volume 2a, so that the operating chamber 3a has the same pressure as the housing volume 2a. In particular, this connection for gas passage is obtained through a wrapper adapted to be passed through by air or another gas and having perforations for example, or preferably made of a gas-permeable material.

In addition, it is made of an elastically deformable material so as to vary its length in response to an outer load. Preferably, it is made of an elastic material characterised by a modulus of elasticity that is not high, preferably of elastan, i.e. a synthetic polyurethane fibre known on the market as Lycra®.

The support 1 has at least connecting means **5** such as a valve or other similar device, adapted to define an opening capable of bringing the housing volume the type taking place upon command and therefore opening and closure of such a connection for gas passage exclusively occurs based on a command given by the user. as utilised in inflatable mattresses or in life buoys or belts. Preferably, the connecting means 5 comprises a nonreturn valve, i.e. a valve adapted to prevent a spontaneous admission of air into the support 1, by maintaining the housing volume 2a hermetically sealed. More specifically, it is adapted to enable entry of gas into the housing volume 2a exclusively in response to a given command or action carried out by the user.

Alternatively, the connecting means 5 may comprise an opening adapted to be connected to a known vacuum machine first enabling air to be extracted from volume 2a and then sealing of the opening thus preventing air passage. In this case the connecting means could be coincident with the orifice 2b.

In detail, the connecting means 5 for command of the operating chamber 3a, enables the housing volume 2a and consequently the volume of the operating chamber 3a to be varied, thus defining an expanded configuration and a compressed configuration of support 1.

In the expanded configuration shown in Fig. 2a, the housing volume 2a substantially has the same pressure as that of the external environment and therefore the deformable support 1 has a maximum extension.

On the contrary, in the compressed configuration shown in Fig. 2b, the housing volume 2a, due to suction of air through the connecting means 5 and inner volume 3, is under vacuum so as to tighten the support 1 on the object 10, as hereinafter described.

In the compressed configuration, since the operating chamber 3a is in connection for gas passage with volume 2a, due to the particular material the inner volume 3 is made of, said chamber tends to become compressed on the product 10 pressing the distinct particles 4 against each other thus preventing them for carrying out a mutual motion. In particular, in this configuration the distinct particles 4 are mutually pressed between the inner volume 3 and the object 10 and therefore they become compacted defining at least one stout or resistant element **4a**, i.e. a substantially stiff body adapted to protect the object 10. In detail, in the compressed configuration several resistant elements having sizes and shapes different from each other can be created, due to formation of several operating chambers 3a suitably filled with the distinct particles 4.

To this aim, the material constituting the distinct particles 4 is a material adapted to enable the particles to adhere to each other when compressed. In detail, this material is preferably a polymer and, more specifically, a thermoplastic polymer.

The resistant element 4a, obtained through compacting of the distinct particles 4, consists of a bladder of distinct particles 4 surrounding the object 10 so as to protect it. In particular, the resistant element is substantially less than 10 cm thick, preferably less than 5 cm, and more preferably less than 3 cm thick.

On the contrary, in the expanded configuration, the operating chamber 3a has the same pressure as the external environment and therefore is unable to press the distinct particles 4 against each other so as to form the resistant element 4a.

In detail, in the expanded configuration, the distinct particles 4 are adapted to take up a volume substantially higher than 50% of the volume taken up by the operating chamber 3a in the expanded configuration. Preferably, said volume of particles 4 is practically higher than 70% and, more preferably, substantially higher than 90% of the volume taken up by the operating chamber 3a in the expanded configuration.

In addition, in order to ensure an appropriate covering of object 10, the distinct particles 4 disposed in an operating chamber 3a are of such a number that they define a resistant element 4a covering at least 50% of the inner surface of the outer wrapper, i.e. the surface of the outer wrapper 2 facing the object 10 when the deformable support 1 is in the compressed configuration.

Said portion of inner surface covered with the resistant element 4a is preferably practically equal to at least 75% and more preferably substantially equal to the whole of said inner surface so as to protect all that part of object 10 that is housed within the deformable support 1.

Operation of the deformable protective support or wrapper described above mainly as regards structure, is the following.

Initially the deformable protective support 1 appears in the expanded configuration, i.e. the outer volume 2 defines the maximum receiving volume 2a. At the moment of use, the user introduces the object 10 into the support 1 through the orifice 2b. In detail, the object 10 during introduction is pressed against the inner wrapper 3 that, due to its ability to be elastically deformed, becomes expanded and wraps the object 10.

In particular, the inner wrapper 3, due to the advantageous difference in volume between the volume defined by the wrapper itself and that taken up by the distinct particles 4, is submitted to extension at the moment the object 10 is introduced. This extension causes arising of return stresses acting on the inner wrapper 3, i.e. forces tending to bring the wrapper 3 back to the compressed configuration.

Once the object 10 has been placed inside the deformable support 1, the user hermetically closes the orifice 2a through the closing means 2c and, if necessary, distributes the distinct particles 4 in a uniform manner around the object 10 through manual pressure exerted on the outer wrapper 2.

When redistribution has been completed, the user through the connecting means 5 (a valve to which a pump is connected, for example) sucks the air from the inside of the deformable support 1 bringing the filling volume 2a and therefore the operating chamber 3a under vacuum pressure, i.e. to a pressure lower than the ambient pressure.

In detail, this operation causes wrappers 2 and 3, due to the vacuum, to tighten against the object 10 thereby tending to compress the distinct particles 4 against each other thus giving rise to formation of at least one resistant element 4a.

When it is wished to extract object 10 from support 1, the user brings the support 1 back to the expanded configuration. The user, through opening of the connecting means 5 or the closing means 2c, makes air enter the housing volume 2a, bringing said housing volume 2a and consequently the operating chamber 3a to the ambient pressure.

In detail, this action causes expansion of the inner wrapper 3 enabling the resistant element 4b to become disgregated since compression of the distinct particles 4 does not occur any longer. These particles are again adapted to be mutually moved causing separation of the resistant element 4a so that the object 10 can be extracted through the orifice 2b.

The invention enables important advantages to be reached.

A first advantage consists in that the deformable protective support or wrapper 1 allows an object 10 to be protected irrespective of its shape.

In fact, the inner wrapper 3 is characterised by high deformability and elasticity, enabling it to adhere to any object 10 in an almost perfect manner.

This advantage is further obtained due to the fact that the inner wrapper 3 in the compressed configuration has an almost smooth contact surface with the object 10.

This aspect is obtained by virtue of the particular volume of the distinct particles 4 in the inner wrapper 3 enabling said wrapper 3 to be tensioned, when the object 10 is housed in the deformable support 1, irrespective of the sizes of the object itself. In fact, introduction of object 10 into the support 1 causes elongation of the inner wrapper 3, as above described, so that said inner wrapper becomes taut and has a substantially smooth surface. Due to this advantage, the deformable support 1 can be also used for protecting parts of a human body such as an arm or leg, for example. In fact, as the inner wrapper 3 defines an almost smooth contact surface, a high comfort sensation is ensured to the user.

Another advantage is represented by the high construction simplicity of the deformable support 1. In fact, housing of the distinct particles 4 inside an operating chamber 3a exclusively made up of the inner wrapper 3 allows both the distinct particles 4 to be placed in a simpler manner inside said chamber and the inner wrapper 2 to be fastened to the outer one 3 without complicated operations being carried out.

A further advantage is due to the great degree of protection ensured by the high strength offered by the resistant element 4a. This advantage is the result of the fact that compression of the distinct particles 4 is due both to the vacuum present within the housing volume 2a and to the above described return tensions exerting a further compacting force on the distinct particles 4. In fact, these return tensions tend to counteract elongation of the inner wrapper so that the distinct particles 4 are made more compact and further pressed together. This protection is further due to the above described almost perfect adhesion between object 10 and inner wrapper 3 allowing the resistant element to adhere to the object 10 in an optimal manner and therefore protect said object in an ideal manner.

Another advantage is represented by the selection of the materials the outer wrapper 2 is made of, which materials enable the object 10 to be isolated from external agents. In detail, these materials allow the contents of the deformable support 1, i.e. object 10, to be isolated from the external environment, so that it is made immune to water, sudden temperature changes and moisture.

Another important advantage is given by the high adaptability of support 1 to the shape of object 10. This advantage is due to the particular material used for manufacturing the inner wrapper 3 that ensures a high deformability of the support 1 and therefore a very high adaptability of the inner wrapper 3, and consequently of the resistant element 4a, to the shape of the object 10, or of an arm of other portion of a human body to be protected.

A still further advantage is the possibility of creating a plurality of chambers 3a due for example to the use of several inner wrappers 3 so that, in the compressed configuration, the deformable support 1 has several resistant elements 4a characterised by different thicknesses and sizes so as to protect also the most brittle and delicate parts of the object 10 in an optimal manner.

## Claims

1. A deformable protective support (1) adapted to at least partly protect an object (10) and comprising an outer deformable and flexible gas-tight wrapper (2) defining a housing volume (2a) suitable to contain at least part of said object (10); at least one inner deformable wrapper (3) adapted to be disposed in contact with at least said part of said object (10); at least one operating chamber (3a) internal to said housing volume (2a) and at least partly defined by said inner wrapper (3); and a plurality of distinct particles (4) housed in said at least one operating chamber (3a); and **characterised in that** said at least one inner wrapper (3) is permeable to gases and defines at least one operating chamber (3a) in connection for gas passage with said housing volume (2a) and **in that** the outer wrapper (2) comprises connecting means (5) adapted to define an opening for bringing said housing volume (2a), upon command, into connection for gas passage with the external environment, in a manner adapted to define an expanded configuration in which said inner volume (2a) substantially has the same pressure as the pressure of said external environment, and a compressed configuration in which said inner volume (2a) has a lower pressure than that of said external environment and wherein said distinct particles (4) are adapted to become compacted so as to define at least one resistant element (4a), which is a substantially stiff body adapted to protect the object (10).

2. A support (1) as claimed in claim 1, wherein said at least one inner wrapper (3) delimits a plurality of operating chambers (3a).

3. A support (1) as claimed in the preceding claim, wherein said inner wrapper (3) consists of a membrane closed upon itself.

4. A support (1) as claimed in the preceding claim, wherein said at least one inner wrapper (3) is made of an elastic material adapted to vary its length when moving between said two configurations.

5. A support (1) as claimed in one or more of the preceding claims, wherein said at least one inner wrapper (3) is adapted to surround a portion of said housing volume (2a) inside it, so as to constitute one said operating chamber (3a).

6. A support (1) as claimed in one or more of the preceding claims, wherein said distinct particles (4) take up a volume greater than 50% of the volume of said operating chamber (3a) when said deformable support (1) is in said expanded configuration.

7. A support (1) as claimed in the preceding claim, wherein said distinct particles (4) take up a volume greater than 90% of said volume of said operating chamber (3a) when said deformable support (1) is in said expanded configuration.

8. A support (1) as claimed in one or more of the preceding claims, wherein said distinct particles (4) are of polymeric material.

9. A support (1) as claimed in one or more of the preceding claims, wherein said resistant element (4a) is adapted to cover at least 50% of the inner surface of said outer wrapper (2) when said deformable support (1) is in said compressed configuration.

10. A support (1) as claimed in the preceding claim, wherein said resistant element (4a) is adapted to substantially cover the whole of said inner surface of said outer wrapper (3) when said deformable support (1) is in said compressed configuration.

11. A support (1) as claimed in one or more of the preceding claims, wherein said outer wrapper (2) comprises closing means (2c) defining an open condition in which said closing means (2c) enables introduction and extraction of said object (10) into and from said housing volume (2a), and a closed condition in which said closing means inhibits passage of said object (10).

12. A support (1) as claimed in the preceding claim, wherein said closing means (2c) is adapted to inhibit gas passage between said housing volume (2a) and the external environment.

13. A support (1) as claimed in the preceding claim, wherein said closing means (2c) comprises an impermeable zip.

14. A support (1) as claimed in one or more of the preceding claims, wherein the connecting means (5) comprises a nonreturn valve adapted to prevent spontaneous admission of gas into said housing volume (2a).

15. Process for at least partly protect an object (10) by means of said deformable protective support (1) as claimed in one or more of the preceding claims, wherein the procedure comprises: the introduction of said object (10) into an orifice (2b) provided in said outer deformable gas-tight wrapper (2) while said protective support is in said expanded configuration, said object (10) being disposed in contact with at least said part of said inner deformable wrapper (3); the hermetically closure of said the orifice (2b) when said object (10) is inside said deformable protective support (1), the suction of the air from the inside of said deformable protective support (1) so that the operating chamber (3a) is under vacuum pressure and said distinct particles (4) are compacted so as to define at least one resistant element (4a), which is a substantially stiff body adapted to protect the object (10).

## Patentansprüche

1. Verformbarer Schutzträger (1), der geeignet ist, einen Gegenstand (10) zumindest teilweise zu schützen und eine verformbare und flexible Außenhülle (2) umfasst, die gasundurchlässig ist und ein Aussparungsvolumen (2) definiert, das geeignet ist, mindestens einen Teil des genannten Gegenstands (10) aufzunehmen; mindestens eine verformbare Innenhülle (3), die geeignet ist, im Kontakt mit mindestens dem genannten Teil des Gegenstands (10) angeordnet zu werden; mindestens eine Arbeitskammer (3a) im Inneren des genannten Aussparungsvolumen (2a), die zumindest zum Teil von der genannten Innenhülle (3) definiert wird; und eine Vielzahl an getrennten Abschnitten (4), die in dieser genannten mindestens einen Arbeitskammer (3a) untergebracht sind; und **dadurch gekennzeichnet sind, dass** diese genannte mindestens eine Innenhülle (3) gasdurchlässig ist und mindestens eine Arbeitskammer (3a) in Verbindung mit dem Gasdurchlass mit diesem Aussparungsvolumen (2a) definiert und dadurch, dass die genannte Außenhülle (2) Verbindungselemente (5) umfasst, die geeignet sind, eine Öffnung zu definieren, um auf Befehl das genannte Aussparungsvolumen (2a) unter Gasdurchlass mit der Außenumgebung so in Verbindung zu bringen, dass eine Ausdehnungskonfiguration definiert wird, bei der das genannten Innenvolumen (2a) einen Druck aufweist, der im Wesentlichen dem Druck der genannten Außenumgebung entspricht und eine Druckkonfiguration, bei der das genannte Innenvolumen (2a) einen Druck unter dem der genannten Außenumgebung aufweist und bei der die genannten getrennten Abschnitte (4) geeignet sind, sich zu verdichten und so mindestens ein Widerstandselement (4a) zu definieren, das im Wesentlichen steif und geeignet ist, den Gegenstand (10) zu schützen.

2. Träger (1) nach Anspruch 1, bei dem die genannte mindestens eine Innenhülle (3) eine Vielzahl von Arbeitskammern (3a) begrenzt.

3. Träger (1) nach dem vorangegangenen Anspruch, bei dem die genannte Innenhülle (3) aus einer in sich selbst geschlossenen Membran besteht.

4. Träger (1) nach dem vorangegangenen Anspruch, bei dem die genannte mindestens eine Innenhülle (3) aus elastischem Material besteht, das geeignet ist, seine Länge während des Durchgangs zwischen den beiden genannten Konfigurationen zu variieren.

5. Träger (1) nach einem oder mehreren der vorangegangenen Ansprüche, bei dem die genannte mindestens eine Innenhülle (3) geeignet ist, in ihrem Inneren einen Teil des genannten Aussparungsvolumen (2a) einzuschließen, um eine genannte Arbeitskammer (3a) zu bilden.

6. Träger (1) nach einem oder mehreren der vorangegangenen Ansprüche, bei dem die genannten getrennten Abschnitte (4) ein Volumen von mehr als 50 % des Volumens der genannten Arbeitskammer (3a) einnehmen, wenn der genannte verformbare Träger (1) sich in der genannten Ausdehnungskonfiguration befindet.

7. Träger (1) nach dem vorangegangenen Anspruch, bei dem die genannten getrennten Abschnitte (4) ein Volumen von mehr als 90 % des genannten Volumens der genannten Arbeitskammer (3a) einnehmen, wenn der genannte verformbare Träger (1) sich in der genannten Ausdehnungskonfiguration befindet.

8. Träger (1) nach einem oder mehreren der vorangegangenen Ansprüche, bei dem die genannten getrennten Abschnitte (4) aus Polymerwerkstoff bestehen.

9. Träger (1) nach einem oder mehreren der vorangegangenen Ansprüche, bei dem das genannte Widerstandselement (4a) geeignet ist, mindestens 50 % der Innenfläche der genannten Außenhülle (2) einzunehmen, wenn der genannte verformbare Träger 1 sich in der genannten Verdichtungskonfiguration befindet.

10. Träger (1) nach dem vorangegangenen Anspruch, bei dem das genannte Widerstandselement (4a) in der Lage ist, im Wesentlichen die Gesamtheit der genannten Innenfläche der genannten Außenhülle (3) einzunehmen, wenn der genannte verformbare Träger (1) sich in der genannten Verdichtungskonfiguration befindet.

11. Träger (1) nach einem oder mehreren der vorangegangenen Ansprüche, bei dem die genannte Außenhülle (2) Verschlussvorrichtungen (2c) umfasst, die eine Öffnungskonfiguration definieren, bei der die genannten Verschlussvorrichtungen (2c) das Einführen und Herausziehen des genannten Gegenstands (10) in und aus dem Aussparungsvolumen (2a) gestatten und eine Schließkonfiguration zu erzielen, bei der die genannten Verschlussvorrichtungen den Durchgang des genannten Gegenstands (10) nicht gestatten.

12. Träger (1) nach dem vorangegangenen Anspruch, bei dem die genannten Verschlussvorrichtungen (2c) geeignet sind, den Gasdurchlass zwischen dem genannten Aussparungsvolumen (2a) und der genannten Außenumgebung zu unterbinden.

13. Träger (1) nach dem vorangegangenen Anspruch, bei dem die genannten Verschlussvorrichtungen (2c) ein undurchlässiges Scharnier umfassen.

14. Träger (1) nach einem oder mehreren der vorangegangenen Ansprüche, bei dem die Verbindungselemente (5) ein Rückschlagventil umfassen, das geeignet ist, einen spontanen Gaseintritt in das genannte Aussparungsvolumen (2a) zu verhindern.

15. Verfahren um einen Gegenstand (10) mittels des genannten Schutzträgers (1) mindestens teilweise zu schützen, wie von einem oder mehreren der vorangegangenen Ansprüche vorgesehen, bei dem das Verfahren das Einführen des genannten Gegenstands (10) in eine Öffnung (2b) auf der genannten verformbaren und gasundurchlässigen Außenhülle (2) umfasst, während der genannte Schutzträger sich in der genannten Ausdehnungskonfiguration befindet, wobei der genannte Gegenstand (10) sich mit mindestens einem Teil der genannten verformbaren Außenhülle (2) in Kontakt befindet; das hermetische Verschließen der genannten Öffnung (2b), wenn der genannte Gegenstand (10) sich im Inneren des genannten verformbaren Schutzträgers befindet, das Ansaugen von Luft im Inneren des genannten verformbaren Schutzträgers (1), so dass in den genannten Arbeitskammern (3a) ein Vakuum vorliegt und die genannten getrennten Abschnitte (4) verdichtet sind, so dass mindestens ein Widerstandselement (4a) definiert wird, das im Wesentlichen aus einem steifen Körper besteht, der in der Lage ist, den Gegenstand (10) zu schützen.

## Revendications

1. Support (1) protecteur déformable apte à protéger au moins partiellement un objet (10) et comprenant une enveloppe externe (2) déformable et souple étanche au passage de gaz et définissant un volume de logement (2a) apte à contenir au moins une partie dudit objet (10) ; au moins une enveloppe interne (3) déformable apte à être mise en contact avec au moins ladite partie dudit objet (10) ; au moins une chambre opérationnelle (3a) à l'intérieur dudit volume de logement (2a) et au moins partiellement définie par ladite enveloppe interne (3) ; et une pluralité de particules (4) distinctes logées dans ladite au moins une chambre opérationnelle (3a) ; et **caractérisé en ce que** ladite enveloppe interne (3) est perméable aux gaz et définit au moins une chambre opérationnelle (3a) en connexion de passage gaz avec ledit volume de logement (2a) et **en ce que** ladite enveloppe externe (2) comprend des moyens de connexion (5) aptes à définir une ouverture pour mettre, sur commande, en connexion de passage gaz ledit volume de logement (2a) avec l'environnement externe de façon apte à définir une configuration d'expansion dans laquelle ledit volume interne (2a) présente une pression fondamentalement égale à la pression dudit environnement externe et une configuration de compression dans laquelle ledit volume interne (2a) présente une pression inférieure audit environnement externe et dans laquelle lesdites particules distinctes (4) sont aptes à se compacter définissant au moins un élément résistant (4a), qui est fondamentalement rigide et apte à protéger l'objet (10).

2. Support (1) selon la revendication 1, dans lequel ladite au moins une enveloppe interne (3) délimite une pluralité de chambres opérationnelles (3a).

3. Support (1) selon la revendication précédente, dans lequel ladite enveloppe interne (3) consiste en une membrane refermée sur elle-même.

4. Support (1) selon la revendication précédente, dans lequel ladite au moins une enveloppe interne (3) est en matériau élastique apte à varier sa longueur durant le basculement entre lesdites deux configurations.

5. Support (1) selon une ou plusieurs des revendications précédentes, dans lequel ladite au moins une enveloppe interne (3) est apte à contenir à son intérieur une partie dudit volume de logement (2a) pour constituer ladite une chambre opérationnelle (3a).

6. Support (1) selon une ou plusieurs des revendications précédentes, dans lequel lesdites particules distinctes (4) occupent un volume supérieur à 50 % du volume de ladite chambre opérationnelle (3a) lorsque ledit support déformable (1) est dans ladite configuration d'expansion.

7. Support (1) selon la revendication précédente, dans lequel lesdites particules distinctes (4) occupent un volume supérieur à 90 % dudit volume de ladite chambre opérationnelle (3a) lorsque ledit support déformable (1) est dans ladite configuration d'expansion.

8. Support (1) selon une ou plusieurs des revendications précédentes, dans lequel lesdites particules distinctes (4) sont en matière polymérique.

9. Support (1) selon une ou plusieurs des revendications précédentes, dans lequel ledit élément résistant (4a) est apte à couvrir au moins 50 % de la surface interne de ladite enveloppe externe (2) lorsque ledit support déformable 1 est dans ladite configuration de compression.

10. Support (1) selon la revendication précédente, dans lequel ledit élément résistant (4a) est apte à couvrir fondamentalement la totalité de ladite surface interne de ladite enveloppe externe (3) lorsque ledit support déformable (1) est dans ladite configuration de compression.

11. Support (1) selon une ou plusieurs des revendications précédentes, dans lequel ladite enveloppe externe (2) comprend des moyens de fermeture (2c) définissant une condition d'ouverture dans laquelle lesdits moyens de fermeture (2c) permettent l'introduction et l'extraction dudit objet (10) dudit volume de logement (2a), et une condition de fermeture dans laquelle lesdits moyens de fermeture empêchent le passage dudit objet (10).

12. Support (1) selon la revendication précédente, dans lequel lesdits moyens de fermeture (2c) sont aptes à empêcher le passage de gaz entre ledit volume de logement (2a) et ledit environnement externe.

13. Support (1) selon la revendication précédente, dans lequel lesdits moyens de fermeture (2c) comprennent une fermeture à glissière étanche.

14. Support (1) selon une ou plusieurs des revendications précédentes, dans lequel les moyens de connexion (5) comprennent un clapet de non retour apte à empêcher une entrée spontanée de gaz dans ledit volume de logement (2a).

15. Procédé pour protéger au moins partiellement un objet (10) par le biais dudit support protecteur (1) comme revendiqué dans une ou plusieurs des revendications précédentes, dans lequel le procédé comprend l'introduction dudit objet (10) dans un orifice (2b) prévu dans ladite enveloppe externe (2) déformable et étanche au passage de gaz alors que ledit support protecteur est dans ladite configuration expansée, ledit objet (10) étant placé en contact avec au moins ladite partie de ladite enveloppe externe (2) déformable ; la fermeture étanche dudit orifice (2b) lorsque ledit objet (10) est à l'intérieur dudit support protecteur déformable, l'aspiration d'air de l'intérieur dudit support protecteur (1) déformable de sorte que lesdites chambres opérationnelles (3a) soient sous vide et lesdites particules distinctes (4) soient compactées de sorte à définir au moins un élément résistant (4a), qui est fondamentalement un corps rigide apte à protéger ledit objet (10).
